(19) Europäisches Patentamt / European Patent Office / Office européen des brevets

(11) **EP 2 640 258 B1**

(12) **EUROPEAN PATENT SPECIFICATION**

(45) Date of publication and mention
of the grant of the patent:
**03.10.2018 Bulletin 2018/40**

(21) Application number: **11788606.9**

(22) Date of filing: **17.11.2011**

(51) Int Cl.:
*A61B 3/107* (2006.01)          *A61B 3/14* (2006.01)
*G06T 7/00* (2017.01)          *G01B 11/25* (2006.01)

(86) International application number:
**PCT/NL2011/050785**

(87) International publication number:
**WO 2012/067508 (24.05.2012 Gazette 2012/21)**

(54) **COLOR CODED TOPOGRAPHER AND METHOD OF DETERMINING A MATHEMATICAL MODEL OF A CORNEAL SURFACE**

FARBKODIERTER TOPOGRAF UND VERFAHREN ZUR BESTIMMUNG EINES MATHEMATISCHEN MODELLS EINER HORNHAUTOBERFLÄCHE

TOPOGRAPHE À CODES COULEUR ET PROCÉDÉ DE DÉTERMINATION D'UN MODÈLE MATHÉMATIQUE D'UNE SURFACE CORNÉENNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.11.2010  NL 2005710
18.11.2010  US 415198 P**

(43) Date of publication of application:
**25.09.2013  Bulletin 2013/39**

(73) Proprietor: **Cassini B.V.
2514 HD 's-Gravenhage (NL)**

(72) Inventor: **MENSINK, Michiel, Herman
2596 TD Den Haag (NL)**

(74) Representative: **EP&C
P.O. Box 3241
2280 GE Rijswijk (NL)**

(56) References cited:
EP-A1- 1 785 690          DE-A1- 4 325 494
DE-C1- 19 752 595          US-A- 4 420 228
US-A- 4 998 819          US-A1- 2006 158 612

**Description**

Technical field:

The present invention relates to a corneal topographer, in particular to a topographer comprising a plurality of light sources of different colors.

Background of the invention:

Corneal topographers are known in general and are used in determining the contour of the anterior surface of the cornea of a human eye, thus facilitating the diagnosis and evaluation of e.g. corneal anomalies, design and fitting of contact lens and the performance of surgical procedures.

Since the anterior surface of the cornea contributes to 2/3 of the refractive power of the eye, it has a major influence in image formation of the eye. The quality of the image formation is dependent on the shape properties of this surface. As such, an important requirement in clinical applications is the accurate assessment of this surface.

The most common method in clinical practice to determine the corneal shape is through a specular reflection technique known as Placido based corneal topography. In this method the reflection of a series of concentric rings is used to determine the shape of the corneal surface. Because the pattern used is a continuous pattern of rings, the analysis for tracing of rays to recover the shape of the cornea is limited to meridian rays. As such, the analysis does not take into account skew rays which can occur if the corneal shape is not rotationally symmetric. As a result, errors in the reconstructed surface may equally occur.

In order to address this, the use of multiple point source corneal topography has been proposed in literature. As an example of such a method, reference can be made to US 4998819 by Labinger et al 1991. In this document, an array of point sources in corneal topography is described whereby the array of sources is described as having the following properties:

1) arranged in a hemispherical mount;
2) the LED pattern is arranged in a semi-meridian. Resulting in bigger spacing between LED points going to the periphery;
3) The LEDs can be designed with colors with suggested groupings of alternate changing in colors.

US 4998819 further describes an algorithm to obtain, from the measured reflections, e.g. on a camera system, a model describing the corneal surface.

It is submitted that the described technology may suffer from the following drawback. In order to provide an accurate model of the corneal shape, it is important to establish the one-to-one correspondence between the point sources applied and the image points, e.g. captured by a camera or camera-lens system.

In order to establish such a one-to-one correspondence, it is suggested in US 4998819 to sequentially pulse the individual light sources. In case of a large number of light sources, this may take a comparatively large amount of time, during which the object that is examined (i.e. the cornea of an eye) should remain stationary.

Further, although this design provides the environment to account for skew ray reflection, it can be noted that the algorithm described is still limited to meridional ray tracing. In meridional ray tracing, the rays are constraint to be lying in a meridian plane. Therefore, the algorithm does not include skew ray reflection.

A device according to the preamble of claim 1 is known from EP 1 785 690 A1. As such, it is an object of the present invention to provide a corneal topographer enabling the one-to-one correspondence between the point sources applied and the image points to be established more easily.

A further object of the present invention is to provide an alternative algorithm for determining a corneal surface model.

Summary of the invention:

According to a first aspect of the present invention, a corneal topographer is provided as defined in claim 1.

In accordance with the first aspect of the present invention, a corneal topographer is provided which comprises a multicolored stimulator. Within the meaning of the present invention, multicolored stimulator is used to denote a light source (not limited to visible light) comprising a plurality of distinct point or point-like sources of different color (in general, sources emitting a different wave length or wavelength spectrum). Such stimulator of point or point-like sources can e.g. be constructed using a plurality of LEDs (e.g. consisting of different groups having different colors) or a plurality of fiber optics.

The plurality of light sources of the stimulator provide a pattern of source points, from which, in use, light rays are provided to an object of interest, e.g. a corneal surface. In case LEDs are used, the source points would thus coincide with the positions of the LEDs.

In a preferred embodiment, the source points are provided on a plurality of boards such as printed circuit boards (PCBs). As an example which is described in more detail below, 7 PCBs are used, each provided with a substantially identical multicolored pattern, e.g. provided by three groups of resp. red, green and blue LEDs.

In accordance with the invention, the plurality of light rays emitted from the source points are in use reflected by the object of interest and received by a lens-camera system of the topographer, thus forming a pattern of image points.

For each of the image points, a computational unit of the topographer is arranged to determine the corresponding source point on the stimulator. This is done, in accordance with the present invention, by using color pattern information that is based on the multicolored pattern of source points of the stimulator and which is e.g. stored in a memory unit of the computational unit. Such color pattern information can e.g. describe, e.g. in array or matrix form, the multicolored pattern or different parts thereof. As an example, the color pattern information can comprise a plurality of 2x2 or 3x3 matrices indicating the colors or different 2x2 or 3x3 sets of source points.

Based on the pattern of image points and the color pattern information, the computational unit of the topographer according to the invention can subsequently establishing a one-to-one correspondence between a source point and an image point. The color pattern information as available to the computational unit can e.g. be applied to recognize similar patterns (e.g. corresponding 2x2 or 3x3 sets of color patterns in both the source points and the image points) enabling a one-to-one correspondence to be established.

In an embodiment, the color pattern information comprises an identifier for each of the source points. Such an identifier can e.g. characterize a source points by its color and one or more colors of the neighboring points. It has been established by the inventors that such a characterization of the source points to a large extend enables the one-to-one correspondence to be established when e.g. 3 different colors of source points are applied.

As such, the identifier (which can e.g. take the form of an array indicating the color of the source point and the colors of neighboring points) is a unique for each source points.

With respect to the prior art described above, US 4998819, it is worth noting that although the use of a multicolored light source is described, this feature is not used in the assessment of the correspondence between the source points and the image points.

In addition, the identifier or color pattern information may also include information with respect to the relative position of the source point in the source point pattern. Combined, the information regarding the relative position and color (i.e. the color of the source point and/or neighboring points) may provide in a unique characterization of the source points and thus enable establishing the one-to-one correspondence.

In a preferred embodiment, the image area can be arranged to be subdivided into several sections; This can e.g. be realized by arranging the source points on a plurality of segments of the multicolored stimulator, e.g. on PCT boards, each segment thus providing in a part of the image area. In such arrangement, a more effective use can be made of using only a limited number of different colors. In such arrangement, the image area sections may be identical or not. In an embodiment, the segments may e.g. have an identical multicolored pattern apart from a unique pattern along one or more edges of the section. The combination of an assessment of the color pattern on the one or more edges with an assessment of a color pattern information as mentioned above, again enables the one-to-one correspondence to be established between the source points and the image points.

In such arrangement, the identification of the image point is thus based on an identifier or color pattern information combined with a further identifier enabling an identification of the segment of the stimulator (e.g. based on a color pattern on an edge).

In general, in accordance with a preferred embodiment of the invention, use is made of an identifier that is based on color pattern information in combination with a further identifier to enable a unique characterization of an image point thus the one-to-one correspondence to be established. Such a further identifier, which e.g. enables an identification of a segment of the stimulator, need not be based on a color pattern of the image points (e.g. the colors of the image points on an edge), but may, as an example, also be based on the observed pattern of image points i.e. the way in which the image points are arranged relative to each other.

By a combined use of a color pattern based identifier and a further identifier, more effective use can be made from a limited number of different colors. Without this, 3 colors would e.g. not be enough to make a unique color coding for e.g. 700 points. Using different sections in the stimulator (each of which can e.g. be characterized / identified by a further identifier (which does not have to be color related)) enables to uniquely identify more image points using only a limited number of colors. Using the one-to-one correspondence, the computational unit can subsequently construct, based on positions of the image points and positions of the corresponding source points, the mathematical model of the surface.

Such construction can be obtained using well-known curve fitting algorithms for surface reconstruction. As an alternative, a surface reconstruction method according to the invention can be applied.

According to a second aspect of the invention, there is provided a method for determining a mathematical reconstruction of a corneal surface, as defined in claim 14.

As will be explained in more detail below, in the present invention, one to one correspondence between image and source points can, to a large extend be established by using color coding of the multiple point source pattern using e.g. three or more source point colors. Further, a systematic mounting configuration for a multicolored stimulator is presented and a new algorithm for accurate surface reconstruction.

These and other aspects of the invention will be more readily appreciated as the same becomes better understood by reference to the following detailed description and considered in connection with the accompanying drawings in which like reference symbols designate like parts.

Brief description of the drawings:

Figure 1 schematically depicts an embodiment of a topographer according to the present invention.

Figure 2 schematically shows a panel comprising a multicolored source point pattern as can be applied in a topographer according to the invention.

Figure 3 schematically shows an image point pattern as obtained using a stimulator comprising 7 panels as shown in Figure 2.

Figure 4 schematically shows part of an image point pattern as can be obtained using a topographer according to the invention.

Description:

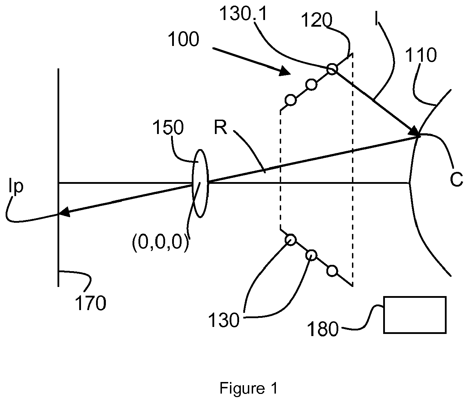

In Figure 1, an embodiment of the corneal topographer according to the present invention is schematically shown. The embodiment schematically shows a multicolored stimulator 100 arranged to project a plurality of light rays onto a corneal surface 110. The stimulator 100 may e.g. have a conical shaped or a hemispherical shaped surface 120 whereby a plurality of the source points are arranged on this surface 120 for projecting the plurality of light rays onto the corneal surface 110. In an embodiment, the source points can be provided by mounting LEDs 130 of different color onto the surface 120. As an alternative, the source points can consist of end-points of fiber optics arranged to emit rays of light towards the corneal surface 110. As schematically shown, an incident ray of light I, emitted by source point (Sp) 130.1 (having coordinates $(X_s, Y_s, Z_s)$) intersects the corneal surface 110 (at corneal intersection point C (having coordinates $(X_c, Y_c, Z_c)$)) and results in a reflected ray of light R towards a lens 150 of a lens-camera system for recording the corneal reflection. The reflected ray of light R is subsequently receive by a camera plane 170 of the lens-camera system resulting in an image point (Ip) having coordinates $(X_i, Y_i, Z_i)$. As can be seen from Figure 1, the opening of the cone 120 (or hemisphere) faces the corneal surface 110 of the eye to be measured. The topographer as shown further comprises a computational unit 180, e.g. a computer, or microprocessor for processing the captured images and construct, based on the captured image, a mathematical representation of the corneal surface 110.

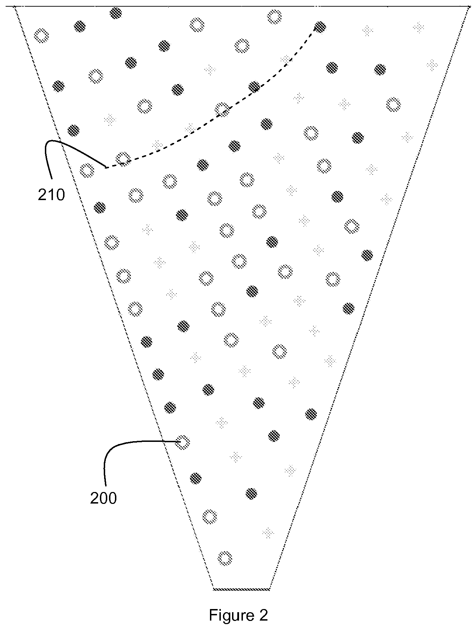

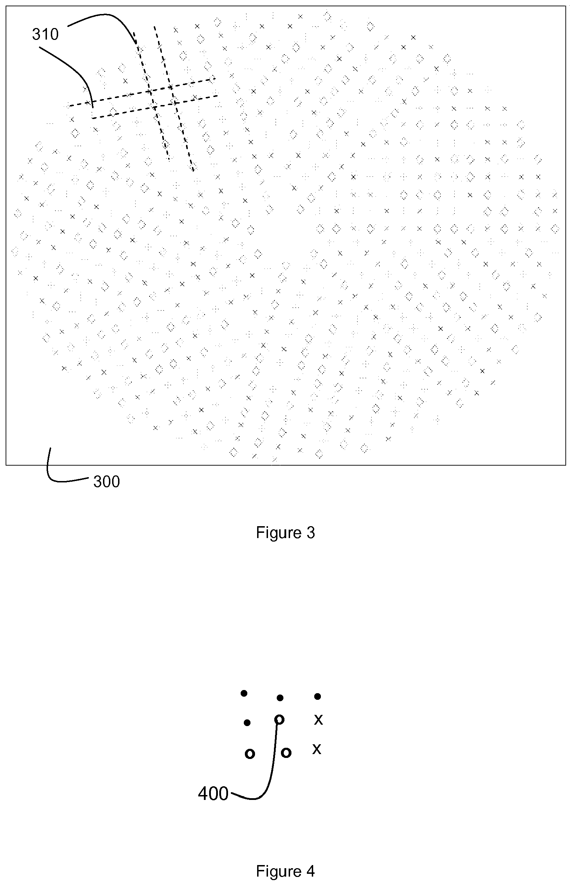

In an embodiment, the multicolored stimulator comprises a plurality of segments, which can be identical or not. As an example of such embodiment, the source points of the stimulator are mounted to a plurality of boards such as PCBs. In such an arrangement, multiple PCB panels can be assembled to form a substantially conic shape. As an example, such a stimulator can be constructed using at least 3 panels. Below, an more detailed example is shown comprising 7 PCB panels. Figure 2 schematically shows a plan view of such a panel 200 whereby the symbols o, + and ● are used to denote source point positions of different color. In the example shown, 96 source points (e.g. LEDs) are arranged on the panel. 7 of such panels, whereby each PCB panel can have an identical arrangement of source points, i.e. colored LEDs, can be assembled to form the substantially conical stimulator. In an embodiment, the arrangement of source point on a panel is such that a reflection on a spherical surface will form a substantially rectangular grid pattern. In figure 3, the resulting image pattern 300 when 7 panels according to Figure 2 are used, is schematically shown. As indicated by the dotted lines 310, the image points obtained form a substantially rectangular grid pattern. In order to identify a particular panel from the other panels (assuming the panels to be identical), the orientation of the rectangular grid pattern can be used. Such an orientation can thus be used to help establishing a unique identification of an image point. The orientation of the grid pattern can e.g. be derived from assessing the positions of a subset of image points. As can be seen from the source point pattern in Figure 2, in order to realise such a rectangular grid pattern as an image point pattern, the source points can be arranged along curved lines, e.g. curvature 210 as indicated in Figure 2.

In order to establish a one-to-one correspondence between the source points and the image points, the corneal topographer according to the invention applies color pattern information based on the multicoloured pattern of source points. Such information can e.g. describe parts of the color pattern, e.g. as a plurality of arrays of matrices. As an example, n x m matrices (e.g. 2x2 or 3x3) can be used to describe the color pattern of n x m source points. By matching the color patterns of the matrices to the pattern of the image points, a correspondence between the image points and the source point can be established. It can further be noted that such pattern matching is facilitated when, as illustrated in Figure 3, the source points of the stimulator are arranged such that a rectangular grid pattern is obtained on the camera-plane. In order to realise this, a predetermined nominal shape of the object of interest can be used to determine how to position the source points on the stimulator in order to obtain the substantially rectangular grid pattern.

As an alternative to characterising the multicoloured pattern of the source points by color pattern information comprising n x m matrices, the source points can be characterised by an identifier. Such an identifier can e.g. comprise a color code based on the color of the source point or neighboring source points.

In an embodiment, in order to facilitate establishing the one-to-one correspondence between the source points and the image points a color coding scheme is employed with the following property: each source point is assigned a color in a manner such that each source point can be characterised by a unique five-color pattern, consisting of its own color and the color of its left, right, up and down neighbours. For the source points on the edge of the panel the absence of a neighbours in one direction can be considered or indexed as another unique color. In order to facilitate the identification of panels, the colors of the source points can be assigned in such a way that the following additional properties are also satisfied:

1) one edge pattern is a unique 2 color combination e.g. RED & GREEN and another edge is another edge pattern is a unique 2 color combination e.g. RED & YELLOW
2) inner ring of the combined panel reflection as received on the camera-plane is arranged to produce one color e.g. GREEN.

When only a limited number of colors available, the number of possible color patterns may not be large enough to assign a unique pattern to each source point. To allow more LEDs than there are unique patterns, the LED assignment property can be relaxed, such that each LED is assigned a five-color pattern (i.e. an identifier) that is only LOCALLY unique. In this case, the LEDs can still be identified if the duplicate patterns are physically separated by sufficient space that there can be no confusion as to which instance of the pattern is being recognized. Phrased differently, apart from the color code of the source point, a relative position or positional information is further used to arrive at determining the one-to-one correspondence between source point and image point. If the panel recognition properties described above are satisfied, this means the duplicate patterns must be separated by at least the maximum amount of displacement of each reflection, which is a property of the maximum aberration that is to be measured.

As an alternative or in addition to using positional information, the identifier of the source points can be detailed further. This is illustrated in Figure 4 showing part of an image point pattern as can be receive by the camera-system of the topographer according to the invention. In Figure 4, an image point 400 having a color indicated by "o" is surrounded by image points having colors "o", "x" or "●". As an identifier for the corresponding source point, an array [o ● x o ●] can be used describing the colors of the source point (first position in the array) and the colors of the source point's up, right, down and left neighbouring point in resp. second third fourth and fifth position of the array. If such characterisation is insufficient for obtaining a unique identifier, the colors of the upper-left, upper-right, lower-right and lower-left neighbour can be used as well, e.g. resulting in an identifier [o ● ● x x o o ● ●] describing the colors of the source point (first position in the array) and the colors of the source point's 8 neighbours in clockwise order starting with the upper neighbour. As will be understood by the skilled person, alternative selections of the number of neighbouring points can be chosen, e.g. depending on the number of colors available, such that a unique identifier can be obtained.

In accordance with the invention, the corneal topographer comprises a computational unit comprising, e.g. in a memory unit, color pattern information (which can e.g. take the form of a table of identifiers as described above or a plurality of matrices describing parts of the source point pattern); the computational unit being arranged to establish the one-to-one correspondence between the image points (as captured by the lens-camera system) and the source points. This can e.g. be done by

- determine an identifier of the image point based on the pattern of image points captured and;
- determining the corresponding source point having the same identifier.

Once this one-to-one correspondence is established, the computational unit can determine a model or mathematical representation of the corneal surface based on the positions of the image points (e.g. obtainable from the captured image on the camera plane 170 as shown in Figure 1 and positions of the corresponding source points, which may be known in advance of determined by calibration of the topographer.

As will be acknowledged by the skilled person, the required complexity of a unique identifier may depend on the color pattern that has been assigned to the pattern of source point. Inversely, starting from a particular type of identifier (e.g. describing the colors of the source point and the colors of the source point's up, right, down and left neighbouring point) the source points need to be arranged in such manner as to substantially allow the one-to-one correspondence being found. In order to determining which colors to assign to the plurality of source points (e.g. LEDs) the following method (which can be automated) can be used:

Step 1: Randomly assign colors to each source point, taking into account any constraints related to panel recognition.
Step 2: Score the color assignment made to the source points. The score is primarily based on the number of unique patterns, but can also include additional desirable properties, such as preferential colors (e.g. to prefer colors for which cheaper LEDs are available with lower power requirements) or patterns that are easier to route on a PCB (e.g. with fewer crossings of power lines to different LEDs).
Step 3: If the score is sufficient, terminate the algorithm, otherwise repeat steps 1 and 2. It has been found that, if the number of possible assignments is sufficiently large (i.e. there are relatively many colors available for the required number of source points), this algorithm terminates quickly on a simple computer.

When a segmented multicoloured stimulator is used, whereby each segment can be identified (e.g. based on an orientation of the image points, or a color scheme used on an edge of the segment) a more efficient use can be made of different colors.

The following table illustrates the number of unique permutations for a given number of colors (n), for internal points and edge points.

Table 1:

| # of colors | # of unique permutations for internal points | # of unique permutations for edge points (e.g. applicable when panel has straight edge boundary) |
| --- | --- | --- |
| 2 | $2^5 = 32$ | $2^4 = 16$ |
| 3 | $3^b = 243$ | $3^4 = 81$ |
| ... | ... | ... |
| n | $n^5$ | $n^4$ |

The table above shows the relationship between the number of colors used in a multicolored stimulator for a corneal topographer and the corresponding unique possible identifiers considering the color of the four nearest neighbor and the color of the point itself. For two colors 32+16 = 48 point grid can still have unique code for each point. By using a plurality of segments (e.g. PCB boards) which can e.g. be identified based on a orientation of the image point pattern observed, The different segments may have identical color patterns, since a unique identification of the image point can be established based on an identification of the image point within a segment (e.g. using the identifier based on the color of the four nearest neighbor and the color of the point itself) combined with an identification of the segment (e.g. based on a further identifier such as an orientation of the image point pattern. If identical color patterns are applied, e.g. on different panels, whereby each panel is assigned a different location and orientation, a further increase in possible identifiers by a color code can be applied at the edges, by considering the absence of a neighbouring point as a further identifier. When considering the position of the missing neighbouring point, this enables to distinguish between one edge of the panel and the opposite edge of the panel. As such, each panel can accommodate at least 32+16+16 = 64 grid points (when at least two colors are used).

As such, in accordance with the invention, a one to one correspondence between source and image points can be realised for any corneal reflection using the color pattern information of the source point pattern. As mentioned, using the rectangular grid property of the image point pattern (when available) may further facilitate establishing the one-to-one correspondence.

In order to construct a mathematical model of the corneal surface (i.e. providing elevation data of the anterior surface of the cornea), the following steps thus need to be taken.

1) Acquire Image

The corneal reflection captured by the camera (e.g. on the image plane 170 of Figure 1) is recorded accordingly. The measurement configuration is taken along the line of sight of the eye. In practice, this can be implemented by making the optical axis of the instrument pass through the centre of the entrance pupil of the eye.

2) Specify location of source and image points

The x,y,z location of the source points can be determined by calibration (e.g. using a modified version of the Labinger et. al. 1991 method as disclosed in US 4998819; the method applying ray tracing taking skew rays into account instead of using meridional ray tracing as done by Labinger). With respect to the position of the image points, the x,y,z location of the centroid of each source point reflection can be considered the position of the image point.

3) One to One correspondence of source and image points

Assuming a source point identifier which describes the colours of the source point and the colours of the source point's up, right, down and left neighbouring point to be available, the following is determined for each source point reflection:

- the colour of the image point,
- the colour coding accounting for the neighbouring image points, i.e. left, right, up and down,
- the PCB panel whereto the source points belongs (provided a plurality of panels is used), and, if required
- the relative location of the source point within the image point pattern, e.g. a rectangular grid pattern.

Using the recorded colour pattern information (i.e. the source point identifiers), one to one correspondence between source and image points is established.

4) Surface Reconstruction e.g. resulting in a Corneal Elevation Map from which other parameters can be derived: keratometric parameters, corneal aberrations, curvature map, residual height maps, etc..)

In accordance with the present invention, the following method is proposed for providing a mathematical model of the corneal surface. It should however be noted that alternative methods known in the art can be applied as well.
As already mentioned above, the corneal surface can produce a distinguishable specular reflection when appropriate light sources are used (e.g. LEDs that produce visible light, IR sources, etc... Depending on the sensitivity of the camera, for example a typical luminous intensity of 50 mcd @ 20mA for a green LED can be used). The modelling of such a reflection phenomenon (as shown in Figure 1) may, in general start with source points having coordinates $xs$, $ys$, $zs$.. These are traced towards the intersection points on the corneal surface $xc$, $yc$, $zc$. and proceed with the reflected ray towards the image points $xi$, $yi$, $zi$. captured by a camera. In general, the reflected ray (R in Figure 1) is constraint to pass through the nodal point 0,0,0,.see Figure 1) of the camera lens system because only the chief ray of the light emitted is considered.
Below, the following definitions and equations will be used:

Table 2 :

| | |
|---|---|
| Reflection source point | $(x_s, y_s, z_s)$ |
| Reflection source point | $(x_i, y_i, z_i)$ |
| | |
| Intersection point on the cornea | $(x_c, y_c, z_c)$ |
| Incedent vector (length x directional cosines) | $\ell_I \langle I_X, I_Y, I_Z \rangle$ |
| Reflected vector (length x directional cosines) | $\ell_R \langle R_x, R_y, R_z \rangle$ |
| Normal vector $N$ on the corneal surface | $\langle N_x, N_y, N_z \rangle$ |

| | | |
|---|---|---|
| Equation of the surface | $-z_c + f(x_c, y_c) = 0$ | (1) |
| Incident Vector | $\langle x_c - x_s, y_c - y_s, z_{c-zs} \rangle = \ell_I \langle I_x, I_y, I_z \rangle$ | (2) |
| Reflected Vector | $\langle -x_c, -y_c, -z_c \rangle = \ell_R \langle R_x, R_y, R_z \rangle$ | (3) |
| Dot Product | $(I + R) \bullet N = 0$ | (4) |
| Cross Product | $(I - R) x N = 0$ | (5) |
| Surface Gradient | $$\langle N_x, N_y, N_z \rangle = \left\langle \frac{\partial f}{\partial x_c}, \frac{\partial f}{\partial y_c}, -1 \right\rangle$$ | (6) |

Given a set of known stimulator source points and corresponding image points, the corneal surface can be reconstructed in the following manner:
The reflection vector as described in equation (3) can be described by:

$$\langle x_i, y_i, z_i \rangle = \ell_R \langle R_x, R_y, R_z \rangle \tag{7}$$

which is derived by tracing from the nodal point to the image point. All parameters in equation (7) are known variables. By combining this with the other equations, an iterative solution to find the appropriate Zernike coefficients describing the corneal surface can be determined using the following procedure. In contrast to alternative methods as known in the art, this new method involves no approximations.

The method can be described as follows:

**STEP 1:** Create matrix equations derived from the equations in Table 1 and Equation (7):

$$\frac{z_c}{r_p} = M\left(\frac{x_c}{r_p}, \frac{y_c}{r_p}\right)C \tag{8}$$

Representing the surface equation expressed in Zernike convention: $p\,r$ is the pupil radius used as a reference for a unit circle, $M$ is the matrix representation of the Zernike polynomials and **C** are the Zernike coefficients. Further, two matrix equations can be derived from the cross product (Equation 5):

$$I_x - R_x = -(I_z - R_z)\frac{\partial M}{\partial x}C \tag{9}$$

$$I_y - R_y = -(I_z - R_z)\frac{\partial M}{\partial y}C \tag{10}$$

**STEP 2:** Use zero initial values for the Zernike coefficients to represent a flat surface in the corneal apex plane. Using ray-tracing, image points can be traced to the apex plane and form the points $xc, yc, zc$.

**STEP 3:** Use $xc, yc, zc.$ as information to calculate a new set of Zernike coefficients **C** by exploiting the matrix equations (8-11). These matrix equations can be cast into the form:

$$A(x_c, y_c, z_c) = B(x_c, y_c, z_c)C \tag{11}$$

The solution for Equation (11) is given by:

$$C = \left[B^T B\right]^{-1}\left[B^T A\right] \tag{12}$$

Which can be solved numerically e.g. using the Moore-Penrose pseudo inverse algorithm.

**STEP 4:** Go back to STEP 2 to build a new surface with the new Zernike coefficients. Proceed to STEP 3 to determine more accurate Zernike coefficients. Repeat this iteration until a desired accuracy has been reached. It has been observed by the inventors that the convergence of the outlined procedure is quite fast. In general, with three iterations a submicron accuracy of the corneal elevation height can already be achieved. In the outlined procedure, the corneal surface is modelled using Zernike polynomials. As will be understood by the skilled person, other conventions can be used, i.e. Taylor polynomials etc. From the basic elevation data which is obtained by the described procedure, the keratometric parameters (axial, local radius of curvature, axis of astigmatism), corneal aberration data, curvature maps and residual height maps can be derived.

The algorithm as described can be compared to the prior art algorithm described by Sicam et al 2004 (J. Opt. Soc. Am. A/Vol. 21, No. 7/July 2004). In the prior art algorithm, an approximation is made to relate two relevant corneal points: 1) the projection of the corneal point from the ray intersection on the corneal apex plane and the 2) the actual ray intersection point (point C in Figure 1) on the cornea. In the new algorithm the former is not necessary anymore for a complete surface reconstruction.

As required, detailed embodiments of the present invention are disclosed herein; however, it is to be understood that the disclosed embodiments are merely exemplary of the invention, which can be embodied in various forms. Therefore, specific structural and functional details disclosed herein are not to be interpreted as limiting, but merely as a basis for the claims and as a representative basis for teaching one skilled in the art to variously employ the present invention in virtually any appropriately detailed structure. Further, the terms and phrases used herein are not intended to be limiting, but rather, to provide an understandable description of the invention.

The terms "a" or "an", as used herein, are defined as one or more than one. The term plurality, as used herein, is defined as two or more than two. The term another, as used herein, is defined as at least a second or more. The terms including and/or having, as used herein, are defined as comprising (i.e., open language, not excluding other elements or steps). Any reference signs in the claims should not be construed as limiting the scope of the claims or the invention.

The mere fact that certain measures are recited in mutually different dependent claims does not indicate that a combination of these measures cannot be used to advantage.

A single processor or other unit may fulfil the functions of several items recited in the claims.

## Claims

1. A corneal topographer comprising:

   a multicolored stimulator (100) comprising a plurality of light sources (130) arranged to form a multicolored pattern of source points (130) for projecting a plurality of light rays onto a surface of an object of interest, such as a cornea (110);
   a lens-camera system (170) arranged to receiving a respective plurality of reflected light rays reflected of the surface of the object of interest, thereby forming a pattern of image points;
   a computational unit (180) for determining a mathematical model of the surface; wherein
   the computational unit comprising a memory unit provided with color pattern information based on the multicolored pattern of source points (130) of the stimulator, the color pattern information comprises a unique identifier for each of the source points,
   the computational unit (180) being arranged to, for each of the plurality of reflected light rays, establishing a one-to-one correspondence between a source point and
   an image point based on the color pattern information;
   the computational unit (180) further being arranged to construct, based on positions of the image points and positions of the corresponding source points, the mathematical model of the surface, **characterised in that** the unique identifier is a unique identifier based on at least a color of neighboring source points of the source point.

2. The corneal topographer according to claim 1 wherein the color pattern information comprises color and relative position information of the source points (130).

3. The corneal topographer according to claim 1, wherein establishing the one-to-one correspondence comprises:

   - determine an identifier of the image point based on the pattern of image points and;
   - determining the corresponding source point having the same identifier.

4. The corneal topographer according to claim 2, wherein establishing the one-to-one correspondence comprises:

   - characterizing each image points based on the image point pattern by its color and a color of neighboring image points and;
   - determining the corresponding source point based on the color and relative position information of the source points.

5. The corneal topographer according to any preceding claim wherein the unique identifier is based on a color of the source point and/or wherein the unique identifier is based on a relative position of the source point in the pattern.

6. The corneal topographer according to any preceding claim whereby the multicolored pattern of source points is provided on a plurality of segments (200).

7. The corneal topographer according to claim 6 wherein a further identifier is applied for identifying a segment of the plurality of segments and wherein establishing the one-to-one correspondence between a source point and an image point is further based on the further identifier.

8. The corneal topographer according to claim 7, wherein the further identifier comprises a color or color pattern applied on an edge of the segment.

9. The corneal topographer according to any of the claims 6 to 8 whereby the plurality of segments comprises a plurality of substantially identical boards such as printed circuit boards (PCBs).

10. The corneal topographer according to claim 9 whereby the multicolored pattern of source points is arranged on the boards to, in use, generate a substantially rectangular grid pattern of image points for each board.

11. The corneal topographer according to claim 10 wherein an orientation of the grid pattern is used as a further identifier for establishing the one-to-one correspondence between a source point and an image point.

12. The corneal topographer according to any preceding claim whereby the multicolored pattern comprises at least three different colors.

13. A method of determining a mathematical model of a corneal surface, the method comprising:

- projecting a plurality of light rays from a multi-colored pattern of source points onto a surface of an object of interest;
- receiving a respective plurality of reflected light rays reflected of the surface of the object of interest thereby forming a pattern of image points;
- providing color pattern information based on the multicolored pattern of source points of the stimulator, the color pattern information comprising a unique identifier for each of the source points, wherein the unique identifier is a unique identifier based on at least a color of neighboring source points of the source point;
- establishing a one-to-one correspondence between the source points and the image points based on the color pattern information;
- determining a mathematical reconstruction of the corneal surface based on a position of the image points and a position of the corresponding source points.

14. The method according to claim 13 wherein the one-to-one correspondence between the source points and the image points is based on an orientation of the image points.

**Patentansprüche**

1. Hornhauttopograph, umfassend:

einem mehrfarbigen Stimulator (100) mit einer Vielzahl von Lichtquellen (130), eingerichtet zum Ausbilden eines mehrfarbiges Musters von Quellenpunkten (130) zum Projizieren einer Vielzahl von Lichtstrahlen auf eine Oberfläche eines Untersuchungsgegenstands, wie einer Hornhaut (110);
ein Linsenkamerasystem (170) eingerichtet zum Empfangen einer jeweiligen Mehrzahl reflektierter Lichtstrahlen, die von der Oberfläche des Untersuchungsgegenstands reflektiert werden, wodurch ein Muster von Bildpunkten gebildet wird;
eine Recheneinheit (180) zum Bestimmen eines mathematischen Modells der Oberfläche;
wobei die Recheneinheit eine Speichereinheit umfasst, die basierend auf dem mehrfarbigen Muster von Quellenpunkten (130) des Stimulators mit Farbmusterinformation versehen ist, wobei die Farbmusterinformation einen eindeutigen Identifikator für jeden der Quellenpunkte umfasst;
wobei die Recheneinheit (180) dazu eingerichtet ist, für jeden der Vielzahl von reflektierten Lichtstrahlen eine Eins-zu-Eins-Entsprechung zwischen einem Quellenpunkt und einem Bildpunkt basierend auf der Farbmusterinformation bereitzustellen;
wobei die Recheneinheit (180) ferner dazu eingerichtet ist, basierend auf Positionen der Bildpunkte und Positionen der entsprechenden Quellenpunkte das mathematische Modell der Oberfläche zu konstruieren,
**dadurch gekennzeichnet, dass**
der eindeutige Identifikator ein eindeutiger Identifikator basierend zumindest auf einer Farbe von benachbarten Quellenpunkten des Quellenpunkts ist.

**2.** Hornhauttopograph nach Anspruch 1, wobei die Farbmusterinformation Farb- und Relativpositionsinformation der Quellenpunkte (130) umfasst.

**3.** Hornhauttopograph nach Anspruch 1, wobei das Bereitstellen der Eins-zu-Eins-Entsprechung umfasst:

- Bestimmen eines Identifikators des Bildpunkts basierend auf dem Muster von Bildpunkten und;
- Bestimmen des entsprechenden Quellenpunkts mit demselben Identifikator.

**4.** Hornhauttopograph nach Anspruch 2, wobei das Bereitstellen der Eins-zu-Eins-Entsprechung umfasst:

- Charakterisieren jedes Bildpunktes basierend auf dem Bildpunktmuster durch seine Farbe und eine Farbe benachbarter Bildpunkte und;
- Bestimmen des entsprechenden Quellenpunkts basierend auf der Farb- und Relativpositionsinformation der Quellenpunkte.

**5.** Hornhauttopograph nach einem der vorhergehenden Ansprüche, wobei der eindeutige Identifikator auf einer Farbe des Quellenpunkts basiert und/oder der eindeutige Identifikator auf einer relativen Position des Quellenpunkts in dem Muster basiert.

**6.** Hornhauttopograph nach einem der vorhergehenden Ansprüche, wobei das mehrfarbige Muster von Quellenpunkten auf einer Vielzahl von Segmenten (200) vorgesehen ist.

**7.** Hornhauttopograph nach Anspruch 6, wobei ein weiterer Identifikator zum Identifizieren eines Segments der Vielzahl von Segmenten angewandt wird und wobei das Bereitstellen der Eins-zu-Eins-Entsprechung zwischen einem Quellenpunkt und einem Bildpunkt ferner auf dem weiteren Identifikator basiert.

**8.** Hornhauttopograph nach Anspruch 7, wobei der weitere Identifikator eine Farbe oder Farbmuster aufweist, das an einer Kante des Segments angeordnet ist.

**9.** Hornhauttopograph nach einem der Ansprüche 6 bis 8, wobei die Mehrzahl von Segmenten eine Mehrzahl von im Wesentlichen identischen Platten umfasst, wie etwa Printed Circuit Boards (PCBs).

**10.** Hornhauttopograph nach Anspruch 9, wobei das mehrfarbige Muster von Quellenpunkten auf den Platten angeordnet ist, um im Betrieb ein im Wesentlichen rechteckiges Gittermuster von Bildpunkten für jede Platte zu erzeugen.

**11.** Hornhauttopograph nach Anspruch 10, wobei eine Orientierung des Gittermusters als ein weiterer Identifikator zum Bereitstellen der Eins-zu-Eins-Entsprechung zwischen einem Quellenpunkt und einem Bildpunkt verwendet wird.

**12.** Hornhauttopograph nach einem der vorhergehenden Ansprüche, wobei das mehrfarbige Muster mindestens drei verschiedene Farben aufweist.

**13.** Verfahren zum Bestimmen eines mathematischen Modells einer Hornhautoberfläche, wobei das Verfahren umfasst:

- Projizieren einer Vielzahl von Lichtstrahlen von einem mehrfarbigen Muster von Quellenpunkten auf eine Oberfläche eines Untersuchungsgegenstands;
- Empfangen einer jeweiligen Mehrzahl reflektierter Lichtstrahlen, die von der Oberfläche des Untersuchungsgegenstands reflektiert werden, wodurch ein Muster von Bildpunkten gebildet wird;
- Vorsehen einer Farbmusterinformation basierend auf dem mehrfarbigen Muster von Quellenpunkten des Stimulators, wobei die Farbmusterinformation einen eindeutigen Identifikator für jeden Quellenpunkt umfasst, wobei der eindeutige Identifikator ein eindeutiger Identifikator basierend auf zumindest einer Farbe von benachbarten Quellenpunkten des Quellenpunktes ist;
- Bereitstellen einer Eins-zu-eins-Entsprechung zwischen den Quellenpunkten und den Bildpunkten basierend auf der Farbmusterinformation;
- Bestimmen einer mathematischen Rekonstruktion der Hornhautoberfläche basierend auf einer Position der Bildpunkte und einer Position der entsprechenden Quellenpunkte.

**14.** Verfahren nach Anspruch 13, wobei die Eins-zu-Eins-Entsprechung zwischen den Quellenpunkten und den Bildpunkten auf einer Orientierung der Bildpunkte basiert.

**Revendications**

1. Topographe cornéen comprenant :

   un stimulateur multicolore (100) comprenant une pluralité de sources lumineuses (130), conçu pour former un motif multicolore de points sources (130) pour la projection d'une pluralité de rayons lumineux sur une surface d'un objet d'intérêt, tel qu'une cornée (110) ;
   un système lentille-caméra (170) conçu pour recevoir une pluralité respective de rayons lumineux réfléchis de la surface de l'objet d'intérêt, formant ainsi un motif de points d'image ;
   une unité de calcul (180) pour la détermination d'un modèle mathématique de la surface ;
   l'unité de calcul comprenant une unité de mémoire approvisionnée en informations de motif de couleur basées sur le motif multicolore de points sources (130) du stimulateur, les informations de motif de couleur comprenant un identifiant unique pour chacun des points sources,
   l'unité de calcul (180) étant conçue pour établir, pour chacun de la pluralité de rayons réfléchis, une correspondance individuelle entre un point de source et un point d'image sur la base des informations de motif de couleur ;
   l'unité de calcul (180) étant en outre conçue pour construire, sur la base des positions des points d'image et des positions des points sources correspondants, le modèle mathématique de la surface, **caractérisé en ce que** l'identifiant unique est un identifiant unique basé sur au moins une couleur de points de source voisins du point de source.

2. Topographe cornéen selon la revendication 1, dans lequel les informations de motif de couleur comprennent les informations de couleur et de position relative des points sources (130).

3. Topographe cornéen selon la revendication 1, dans lequel l'établissement de la correspondance individuelle comprend :

   - la détermination d'un identifiant du point d'image sur la base du motif de points d'image ; et
   - la détermination du point de source correspondant ayant le même identifiant.

4. Topographe cornéen selon la revendication 2, dans lequel l'établissement de la correspondance individuelle comprend :

   - la caractérisation de chaque point d'image sur la base du motif de points d'image par sa couleur et une couleur des points d'image voisins ; et
   - la détermination du point de source correspondant sur la base des informations de couleur et de position relative des points sources.

5. Topographe cornéen selon l'une des revendications précédentes, dans lequel l'identifiant unique est basé sur une couleur du point de source et/ou dans lequel l'identifiant unique est basé sur une position relative du point de source dans le motif.

6. Topographe cornéen selon l'une des revendications précédentes, le motif multicolore de points sources étant disposé sur une pluralité de segments (200).

7. Topographe cornéen selon la revendication 6, dans lequel un identifiant supplémentaire est appliqué pour identifier un segment de la pluralité de segments et dans lequel l'établissement de la correspondance individuelle entre un point de source et un point d'image est en outre basé sur l'identifiant supplémentaire.

8. Topographe cornéen selon la revendication 7, dans lequel l'identifiant supplémentaire comprend une couleur ou un motif de couleurs appliqué sur un bord du segment.

9. Topographe cornéen selon l'une des revendications 6 à 8, la pluralité de segments comprenant une pluralité de cartes globalement identiques tels que des cartes de circuits imprimés (PCB).

10. Topographe cornéen selon la revendication 9, le motif multicolore de points sources étant disposé sur les cartes afin de générer, lors de l'utilisation, un motif de grille globalement rectangulaire de points d'image pour chaque carte.

11. Topographe cornéen selon la revendication 10, dans lequel une orientation du motif de grille est utilisée en tant qu'identifiant supplémentaire pour l'établissement de la correspondance individuelle entre un point de source et un point d'image.

12. Topographe cornéen selon l'une des revendications précédentes, le motif multicolore comprenant au moins trois couleurs différentes.

13. Procédé de détermination d'un modèle mathématique d'une surface cornéenne, ce procédé comprenant :

- la projection d'une pluralité de rayons lumineux à partir d'un motif multicolore de points sources vers une surface d'un objet d'intérêt ;
- la réception d'une pluralité respective de rayons lumineux réfléchis de la surface de l'objet d'intérêt, formant ainsi un motif de points d'image ;
- la fourniture d'informations de motif de couleurs basées sur le motif multicolore de points sources du stimulateur, les informations de motif de couleurs comprenant un identifiant unique pour chacun des points sources, l'identifiant unique étant un identifiant unique basé sur au moins une couleur de points sources voisins du point de source ;
- l'établissement d'une correspondance individuelle entre les points sources et les points d'image sur la base des informations de motif de couleurs ;
- la détermination d'une reconstruction mathématique de la surface cornéenne sur la base d'une position des points d'image et d'une position des points sources correspondants.

14. Procédé selon la revendication 13, dans lequel la correspondance individuelle entre les points sources et les points d'image est basé sur une orientation des points d'image.

Figure 1

210

200

Figure 2

Figure 3

Figure 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4998819 A, Labinger

- EP 1785690 A1

**Non-patent literature cited in the description**

- **SICAM et al.** *J. Opt. Soc. Am. A,* July 2004, vol. 21 (7